# FASCICULE DE BREVET EUROPEEN

(11) **EP 0 244 326 B1**
(45) Date de publication et mention de la délivrance du brevet: **18.08.1993**
(21) Numéro de dépôt: 87401000.2
(22) Date de dépôt: 30.04.1987
(51) Int. Cl.: G01N 33/48

(54) **Procédé de détection et/ou d'indentification d'une substance biologique dans un milieu liquide à l'aide de mesures électriques, et dispositif destiné à la mise en oeuvre de ce procédé**
Verfahren zum Feststellen und/oder zum Identifizieren einer biologischen Substanz durch elektrische Messungen und Vorrichtung zum Durchführen dieses Verfahrens
Method for detecting and/or identifying a biological substance in a liquid medium with the aid of electrical measurements, and apparatus for carrying out this method

(30) Priorité: 30.04.1986 FR 8606315
(43) Date de publication de la demande: 04.11.1987
(73) Titulaire: BIO MERIEUX Société anonyme dite:, F-69260 Charbonnieres les Bains (FR)
(72) Inventeur: Colin, Bruno, F-69660 Collonges au Mont d'Or (FR); Mandrand, Bernard, F-69100 Villeurbanne (FR); Martelet, Claude, F-69370 St Didier au Mont d'Or (FR); Jaffrezic, Nicole, F-69130 Ecully (FR)
(74) Mandataire: Nony, Michel

(56) Documents cités:
- EP-A- 0 121 385
- GB-A- 2 136 130
- US-A- 4 490 216

## Description

La présente invention a pour objet un procédé de détection et/ou d'identification d'une substance biologique dans un milieu liquide à l'aide de mesures électriques, ainsi qu'un dispositif destiné à la mise en oeuvre de ce procédé.

On sait que la détection de substances biologiques est le plus souvent effectuée à l'aide de réactifs doués d'affinité pour ces substances. En particulier, les méthodes fondées sur la réaction antigène-anticorps sont bien connues. Généralement ces méthodes nécessitent des temps d'incubation importants et la révélation de la réaction est effectuée à l'aide de marqueurs de coût élevé et de manipulation délicate, tels que des marqueurs radioactifs ou enzymatiques.

On a également proposé de détecter la réaction antigène-anticorps en fixant les anticorps sur une membrane et en utilisant les variations de différence de potentiel provoquées par ladite réaction à l'interface membrane-solution pour faire varier le champ électrique transversal au voisinage d'un transistor à effet de champ. Il en résulte une variation de la résistivité entre drain et source du transistor que l'on peut mettre en évidence par une mesure potentiométrique. Toutefois, de tels systèmes se sont avérés non spécifiques ; voir par exemple J. JANATA et al., Annals New York Academy of Sciences, 286-292 (1984).

Le document GB-A- 2.136.130 décrit un dispositif en couche mince, comprenant des électrodes déposées sur un support isolant et recouvertes d'une couche de matériau diélectrique. Sur la surface libre de la couche de diélectrique, et donc parallèlement au champ électrique, on dépose un réactif capable de réagir avec un partenaire chimique ou biologique éventuellement présent dans un liquide à étudier. Pour la mise en oeuvre, on dépose une goutte du liquide étudié sur la couche de réactif et on note la variation de capacité du système d'électrodes. Il s'agit donc d'un système fonctionnant à sec, sans électrode au contact du liquide. La modification de capacité est due à un effet latéral ou effet de bord, et la présence du partenaire chimique ou biologique se traduit pas une augmentation de capacité.

Le brevet US 4.490.216 décrit un dispositif sensible aux modifications de polarité, comprenant un support conducteur recouvert sur une face d'une couche isolante elle-même surmontée d'une bicouche lipidique. La couche lipidique externe est liée à un réactif tel qu'un anticorps. Pour rechercher si un liquide contient un partenaire dudit réactif, on met en contact le dispositif avec le liquide, et on note la modification éventuelle du potentiel entre le support conducteur et une électrode de référence.

On a maintenant découvert qu'il est possible de détecter une réaction d'affinité du type ligand-substance biologique par un procédé simple et sensible fondé sur des mesures d'impédance électrique du circuit complexe formé par une solution contenant la substance biologique étudiée, une couche mince isolante sur laquelle est fixée le ligand, et un support conducteur sur lequel est fixé l'isolant.

L'invention a donc pour objet un procédé de détection d'une substance biologique dans un milieu liquide conducteur de l'électricité susceptible de la contenir, caractérisé par le fait que l'on met en contact, dans une cellule de mesure, ledit milieu liquide avec une plaque porte-réactif comportant un ligand spécifique fixé directement sur une face d'une couche mince en matériau isolant dont l'autre face est fixée sur un support capable de conduire l'électricité, que l'on mesure les composantes C (capacité) et/ou R (résistance) de l'impédance électrique du système milieu liquide/couche isolante/support pour une tension donnée, et que l'on compare lesdites composantes à celles d'un système de référence analogue au précédent mais exempt de ligand spécifique, une éventuelle diminution de la capacité ou une variation de la résistance, par rapport au système de référence, signifiant la présence de ladite substance biologique dans le milieu liquide étudié.

Généralement, il suffit de mesurer la capacité électrique du système mais dans certains cas la mesure de résistance est plus sensible.

Le procédé de l'invention s'applique à toute substance biologique capable d'être reconnue par un ligand doué d'affinité pour ladite substance.

Parmi les couples ligand-substance biologique, on citera par exemple les couples anticorps-antigène, haptène-anticorps, ADN_{c}-ADN, ADN_{c}-ARN, poly dT-ARNₘ eucaryote, enzyme-substrat, enzyme-inhibiteur d'enzyme, lectine-glycoconjugué, marqueur de cellules (ou de microorganismes)-cellules (ou microorganisme), HcG-récepteur tissulaire, et T₃-TBG (thyroxin binding globulin) et, d'une façon générale, les couples récepteur biologique-ligand correspondant (ou agoniste ou antagoniste). Selon les cas, l'un ou l'autre élément du couple pourra être fixé sur la couche isolante.

Le ligand peut également être un marqueur de cellules ou de microorganismes, par exemple un marqueur du typage cellulaire, la substance biologique étant alors constituée par les cellules ou microorganismes que l'on souhaite identifier ou dont on recherche le type.

Dans la suite de la description, on fera souvent référence, par commodité, au système antigène-anticorps. Cette simplification est purement conventionnelle et ne doit bien entendu pas être considérée comme limitative.

La capacité ou la résistance mesurée sera fonction de l'épaisseur (fixe) de la couche d'isolant et de l'épaisseur et de la densité (variable) de la couche de ligand et de substance biologique fixés.

La sensibilité du système est bien entendu fonction des dimensions relatives de la couche isolante (épaisseur) et de la substance biologique étudiée. Il est clair en outre que le ligand fixé sur la couche isolante doit avoir les dimensions les plus faibles possible.

Au contraire, les substances destinées à être fixées sur le système isolant-ligand doivent avoir les dimensions les plus grandes possible, et on peut dans certains cas augmenter la sensibilité de la détection à l'aide de divers artifices basés sur ce principe. Par exemple, lorsque la substance biologique étudiée comporte plusieur sites d'affinité, on peut ajouter au liquide étudié, après mise en contact dudit liquide avec la plaque porte-réactif, un second ligand qui viendra se fixer à son tour sur les sites d'affinité encore libres de ladite substance biologique, si elle est présente, en augmentant ainsi l'épaisseur des couches déposées sur l'isolant. Le second ligand peut être identique ou non au ligand fixé sur l'isolant.

On peut encore augmenter la sensibilité en fixant le second ligand sur une macromolécule telle qu'une protéine ou sur de petites particules individuelles capables de se maintenir en suspension dans le milieu liquide. Ces particules sont par exemple des particules solides (latex, polystyrène, et...) ou des microglobules tels que des liposomes.

Le liquide conducteur peut être toute solution tampon compatible avec le système biologique utilisé. Ce milieu liquide peut avoir une conductivité équivalente à celle d'une solution aqueuse de chlorure de sodium ayant une concentration pouvant aller de 0,015M à 3M. Le pH du milieu liquide peut être compris entre 2 et 11. Les interactions non spécifiques par échange d'ions ou par interactions hydrophobes peuvent être combattues par des tampons de force ionique appropriée contenant éventuellement des détergents utilisés classiquement à cet effet.

Pour mesurer les composantes R et C du système obtenu on met en contact une électrode inattaquable avec le liquide étudié et l'on établit une tension alternative entre ladite électrode et ledit support conducteur. On détermine la capacité et/ou la résistance du système à une tension donnée selon les méthodes connues. On compare ensuite avec la grandeur correspondante mesurée avec le système de référence.

On applique en outre une tension de polarisation, de l'ordre de quelques volts au maximum, au support conducteur de la plaque porte-réactif, par rapport à une électrode de référence, par exemple une électrode au calomel. On fait généralement varier la tension de polarisation (positive ou négative) et on détermine ainsi facilement la tension de polarisation qui permet d'observer les variations optimales des composantes R ou C.

Généralement, les tension optimales de polarisation, pour la détermination de la composante C, varient de 1,5 à 5 volt, le plus souvent de 3 à 5 volts. On observe toujours une diminution de la capacité.

Pour la détermination de la composante R, les tensions de polarisation optimales sont voisines de la tension nulle, généralement entre - 1 volt et + 1 volt. Les variations de la résistance dépendent du système réactif-antigène utilisé. Avec certains systèmes, on observe une augmentation de la résistance, et une diminution avec d'autres systèmes.

La mesure peut être réalisée, pour la détection des protéines, par fixation directe sur l'anticorps spécifique lui-même fixé sur la phase solide, avec ou sans amplification. On peut avantageusement utiliser des fragments Fab à la place des anticorps entiers, les fragments Fab étant préparés par exemple selon une méthode analogue à celle décrite par E. Ishikawa, J. Immunoassays, 4, 209 (1983).

Pour mesurer une concentration de petite molécule (haptène) la méthode par compétition peut être préférable, en particulier en fixant l'haptène sur la phase solide par l'intermédiaire d'un agent chimique de couplage et en mesurant la fixation de l'anticorps spécifique, d'autant plus importante que l'échantillon est plus pauvre en molécule à doser.

La méthode inverse avec un anticorps fixé et un haptène lié à une molécule ou une particule de grosse taille peut également être utilisée.

Pour la recherche ou le typage cellulaire, la méthodologie présentée est particulièrement attractive du fait de la taille importante des cellules qui, en se fixant, vont entraîner une chute très forte de la capacité mesurée.

Les domaines d'application recouvrent donc notamment l'ensemble des déterminations immunologiques sur cellules, protéines ou haptènes. La surface active est récupérable pour une nouvelle mesure après lavage avec un agent dissociant du complexe formé, par exemple par lavage acide puis équilibrage.

Le système se prête bien à des déterminations multiples sur un seul échantillon, que ce soit en recherche d'anticorps, d'antigène ou d'haptène ou en typage cellulaire. Toutes ces déterminations peuvent être effectuées simultanément en quelques minutes avec une surface portant autant de plaquettes greffées que de déterminations souhaitées, une ou plusieurs plaquettes témoins fixant le niveau du bruit de fond.

Le dispositif peut être plan ou cylindrique avec un volume d'échantillon éventuellement très limité.

Un exemple d'un tel dispositif peut être fourni par un système de recherche des microorganismes pathogènes dont la présence est éventuellement attendue sur un échantillon résultant d'un prélèvement de selles ou d'un écouvillonnage.

Le système de mesure capacitif est par ailleurs utilisable à température très élevée, ce qui permet d'optimiser la température réactionnelle.

Un exemple très important d'application est constitué par l'hybridation de sondes nucléiques. Du fait de la taille des ADN et ARN, plus élevée que celle des protéines correspondantes, une chute capacitive importante sera mesurée après une hybridation entre un oligonucléotide fixé sur une plaquette de silice et l'acide nucléique portant la séquence complémentaire.

L'ensemble du système de mesure résistant à des températures élevées voisines de 95°C, la totalité du processus de recherche des séquences nucléiques spécifiques pourra être réalisée dans la même cuve. Le chauffage est effectué d'abord pour déshybrider ; ensuite la réaction spécifique peut être suivie par abaissement régulier de la température.

Après élévation de la température de la cuve, au-dessus de la température critique d'hybridation, et lavage à force ionique faible, la cuve est utilisable à nouveau pour une détermination nouvelle.

Comme dans le cas des systèmes immunologiques, le signal obtenu peut être amplifié par une deuxième sonde nucléique utilisée dans la phase liquide, seule ou liée à une macromolécule ou une particule de taille importante.

Le procédé de l'invention permet d'effectuer non seulement des déterminations qualitatives, mais aussi des déterminations quantitatives. Pour cela, il suffit d'étalonner la cuve de mesure, munie de plaques porte-réactif normalisées, avec des quantités connues croissantes de la substance biologique que l'on veut détecter ou identifier.

L'invention a également pour objet une plaque porte-réactif destinée à la mise en oeuvre du procédé décrit ci-dessus.

Cette plaque porte-réactif est caractérisée par le fait qu'elle comporte un support en matériau capable de conduire l'électricité, recouvert sur une de ses faces par une couche mince isolante sur laquelle est fixé directement un ligand pour une substance biologique.

Dans des modes de réalisation particuliers, la plaque porte-réactif peut encore présenter les caractéristiques suivantes :
- le matériau capable de conduire l'électricité est un métal et la couche isolante est une couche d'oxyde de ce métal ; le métal est par exemple l'aluminium, le tantale, etc...
- ou bien le support est constitué par un matériau semi-conducteur et il comporte, sur la face opposée à la face recouverte de la couche isolante, une couche métallique destinée à assurer un contact ohmique ; le semi-conducteur est par exemple le silicium et la couche isolante est une couche de silice ; (SiO₂) ou de nitrure de silicium (Si₃N₄).
- le support a généralement une forme plane ; il peut aussi avoir par exemple une forme tubulaire ou une forme de coupe hémisphérique ;
- le ligand est fixé sur la couche isolante par adsorption, ou bien il est greffé sur la couche isolante par covalence.

Pour préparer une plaque porte-réactif on peut par exemple l'oxyder superficiellement en atmosphère d'oxygène à température convenable. L'épaisseur de la couche d'oxyde isolante peut être facilement réglée, par exemple en faisant varier le temps de réaction pour une température réactionnelle donnée.

De préférence on utilise des plaques dont la couche isolante a une épaisseur inférieure à 0,1 µm.

Le ligand peut être fixé par adsorption sur la couche isolante ; on connaît par exemple les propriétés adsorbantes de l'alumine ou de la silice qui sont largement utilisées en chromatographie.

On peut également greffer le ligand par covalence en utilisant un réactif de couplage bifonctionnel capable de réagir à la fois avec la couche isolante et avec le ligand. L'utilisation de tels réactifs est bien connue.

Par exemple, dans le cas de la silice, on peut greffer par covalence les molécules aminées, y compris les protéines à l'aide d'un dérivé de silane de formule :

(Alk O)₃ Si - X - R

dans laquelle
- Alk représente un alkyle inférieur,
- X représente un bras hydrocarboné tel qu'un alkylène inférieur
   et
- R est un groupement réactif tel qu'un groupement NH₂.

Ce composé réagit avec la silice pour donner une silice substituée, par exemple du type : 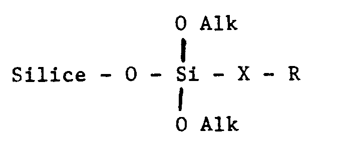
On peut alors facilement fixer une molécule aminée, sur la silice ainsi modifiée, à l'aide d'un réactif bifonctionnel qui établit des liaisons à la fois avec le groupement réactif R et avec les groupement NH₂ du ligand. Par exemple lorsque R représente NH₂, on peut utiliser un dialdéhyde. Le groupement carbonyle réagit avec le groupement NH₂ pour former une imine qui peut être réduite, si désiré, en groupement aminé correspondant, par exemple à l'aide d'un borohydrure.

Une telle méthode de greffage sur la silice est décrite notamment par P.J. ROBINSON et coll., Biochimica et Biophysica Acta 242, 659-661 (1971).

D'autres méthodes de greffage sur silice qui peuvent être utilisée sont celles décrites notamment par M.K. WEIBEL et coll., Biotechnology and Bioengineering, Vol.XVII, 85-98 (1975) ; Y. HAMAGUCHI et coll., Eur. J. Biochem. 71, 459-467 (1976) ; H.H. WEETALL et coll., Biotechnology and Bioengineering, Vol. XVIII, 105-118 (1976) ; et H.D. CRONE et coll., Journal of Chromatography, 129, 91-96 (1976).

L'invention concerne également une cuve de mesure destinée à mettre en oeuvre le procédé défini ci-dessus. Cette cuve de mesure est principalement caractérisée par le fait qu'elle comprend une enceinte comportant un espace interne pour l'échantillon liquide, un réceptacle pour une plaque porte-réactif amovible, permettant de mettre en communication la face interne, portant la couche isolante munie du ligand, de ladite plaque, avec l'intérieur de l'enceinte, et éventuellement un second réceptacle pour une plaque de référence sans ligand spécifique, des moyens pour assurer l'étanchéité entre l'enceinte et lesdites plaques, une électrode susceptible d'être en contact avec ledit échantillon liquide, et des moyens pour assurer des contacts électriques extérieurs pour l'électrode et pour la face externe des plaques.

L'invention va maintenant être décrite plus en détail en faisant référence aux dessins annexés dans lesquels :
- la figure 1 est un schéma fonctionnel d'un système de mesure permettant la mise en oeuvre du procédé de l'invention et
- les figures 2 et 3 représentent des vues schématiques en coupe de modes de réalisation particuliers de la cuve de mesure ;

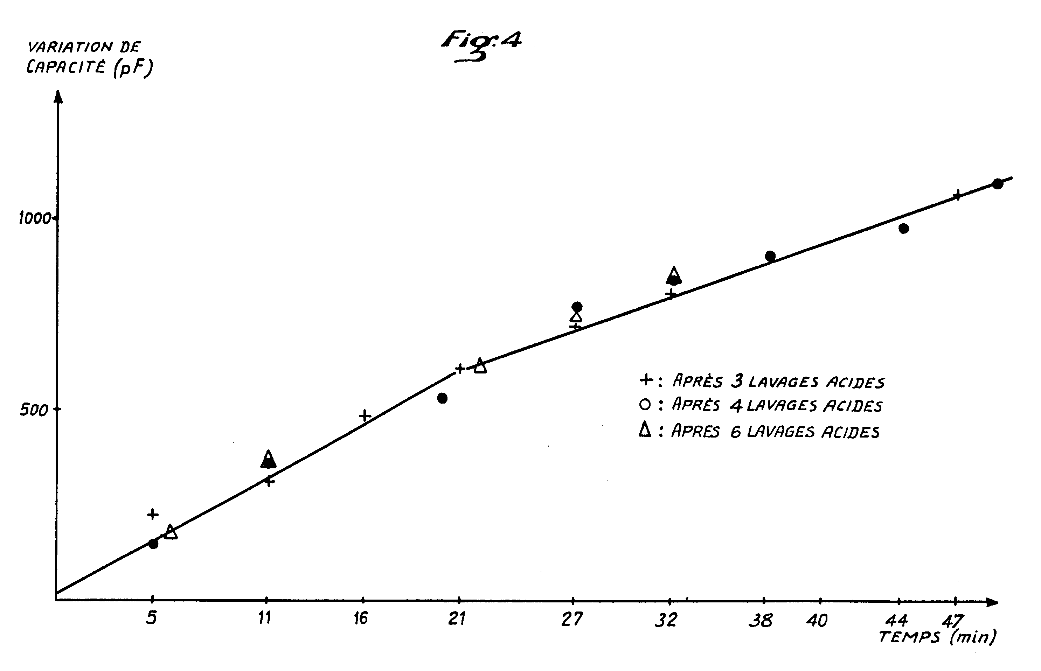

- la figure 4 représente la variation de capacité en fonction du temps dans les conditions indiquées à l'exemple 3 ;
- et la figure 5 représente les variations de capacité observées, en fonction de la concentration en protéine, avec deux plaques porte-réactif préparées de façon identique (voir exemple 3).

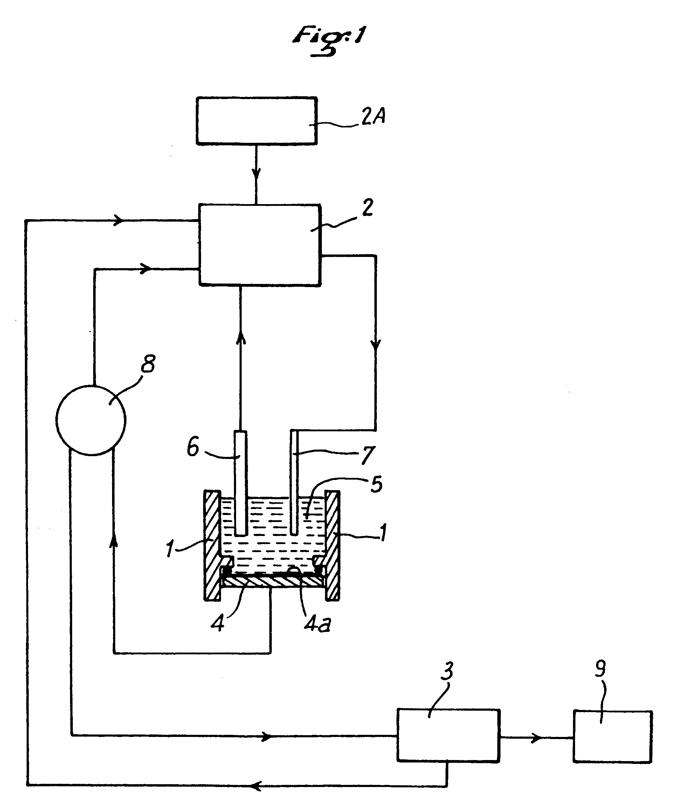

Le dispositif complet comprend une cuve de mesure 1, un module d'alimentation en tension 2 (potentiostat) réglable commandé par un calculateur 3. La plaque porte-réactif 4 servant d'électrode de travail est par exemple une plaquette de silicium. Sur la face supérieure de cette plaquette (figure 1), le trait épaissi 4a représente la couche de silice sur laquelle ont été greffés par exemple des anticorps. L'échantillon liquide 5 présent dans la cuve de mesure est en contact avec cette couche de silice munie du ligand. Le contact ohmique de la face inférieure de la plaquette 4 est réalisé par une couche d'or non représentée, obtenue par évaporation sous vide.

Une électrode 6 (électrode au calomel par exemple) qui sert d'électrode de référence, est reliée au potentiostat 2, assurant son asservissement et donc une polarisation bien définie de la plaquette de silicium par rapport à l'échantillon liquide. Un tel système est nécessaire dans le cas d'une plaquette en semi-conducteur afin d'imposer à celui-ci une une tension de polarisation suffisante, supérieure au travail de sortie, permettant de rendre ce matériau conducteur.

Le potentiostat 2 est également relié à une contre-électrode en platine 7 et permet d'appliquer à cet électrode une tension alternative dont la fréquence peut varier par exemple depuis 1 kHz jusqu'à 1 mégaherz. Le potentiostat est alimenté par le générateur 2A. L'analyse du signal fait intervenir un modèle mathématique de circuit RC. On peut donc effectuer à la fois une mesure de la résistance et une mesure de capacité pour la même tension appliquée. L'analyse du signal retour par le module de mesure du courant 8 permet de connaître les composantes R et C de l'impédance du système qui sont transmises au calculateur 3. Les résultats sont transcrits par l'imprimante 9.

La tension alternative appliquée (qui se superpose à la tension de polarisation éventuellement appliquée à la plaque porte-réactif) peut avoir une amplitude allant de 1 millivolt à quelques volts. Cette tension doit être compatible avec la stabilité du milieu liquide et des substances biologiques présentes.

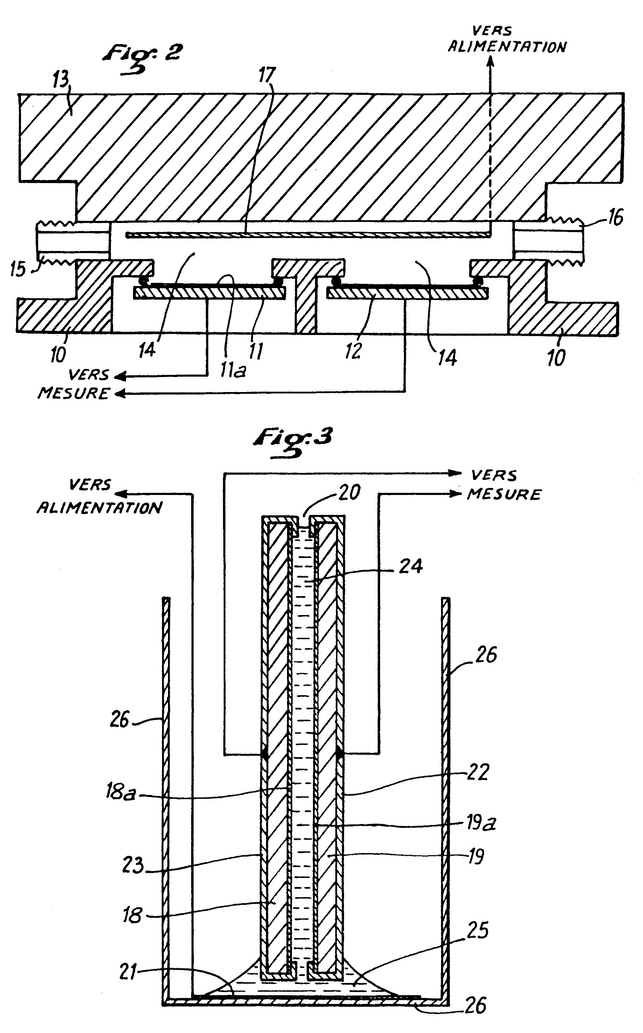

La cuve de mesure de la figure 2 est constituée par exemple d'un bloc isolant en polycarbonate dont la partie inférieure 10 comporte deux logements respectivement pour la plaque porte-réactif 11 et la plaque de référence 12. Les dispositifs de fixation des plaques 11 et 12 n'ont pas été représentés. La partie supérieure 13 peut être thermostatée.

Entre les parties inférieure et supérieure est ménagé un espace creux 14 pour l'échantillon liquide, avec un orifice d'entrée 15 et un orifice de sortie 16. Dans l'espace interne creux 14 est disposée une électrode en platine 17. La face supérieure de la plaquette 11, destinée à être en contact avec l'échantillon liquide à étudier, est constituée par une couche mince de silice sur laquelle est greffé le ligand spécifique et la face supérieure de la plaque de référence 12 est constituée par une couche mince de silice sans ligand spécifique. De préférence, la plaque de référence porte une couche monomoléculaire de même nature (par exemple une immunoglobuline non spécifique) que le ligand de la plaquette 11. Par comparaison des composantes R et C obtenues avec les plaquettes 11 et 12, ce dispositif permet d'effectuer une mesure différentielle et d'éliminer ainsi les effets d'une éventuelle adsorption non spécifique.

Le dispositif de la figure 3 représente schématiquement, en agrandissement, une cellule de mesure miniaturisée.

Dans ce dispositif, la plaque porte-réactif 18 avec la couche isolante 18a munie de réactif (ligand spécifique) et la plaque de référence munie de la couche de silice 19a, éventuellement revêtue d'une molécule non spécifique de même nature que le ligand spécifique, sont disposées en regard l'une de l'autre ; elles sont séparées par un espace étroit 20, dans lequel une fraction 24 de la goutte d'échantillon liquide 25 déposée sur le fond de la cuve 26 peut s'insérer et être retenue par capillarité. Bien entendu, les dimensions relatives de l'espace 20 ont été agrandies pour la clarté du dessin. A l'extrémité inférieure, la base de la goutte liquide est en contact avec une électrode en platine représentée par un trait épaissi 21. La face externe des plaques 18 et 19 est dans ce cas revêtue d'un vernis isolant 22, 23. On peut ainsi faire des mesures sur des échantillons de quelques microlitres.

### EXEMPLE 1 : Obtention d'une plaque porte-réactif

On utilise des plaquettes de silicium dopées de type p ayant 6cm de diamètre et 0,2 mm d'épaisseur, commercialisées par la société CSEM (Neufchatel, Suisse).

Par oxydation en atmosphère d'oxygène à 1200°C pendant 24heures d'une des faces de la plaquette, on obtient une couche de silice ayant une épaisseur de 700 Angströms.

L'autre face est rendue conductrice par métallisation par évaporation d'or sous vide.

Après hydratation de la couche de silice pendant 48heures en atmosphère de vapeur d'eau à 100°C, on immerge les plaquettes dans une solution de 3-aminopropyltriéthoxysilane à 2% dans l'acétone pendant 24heures à 45°C.

On lave les plaquettes à l'acétone et on sèche.

On immerge ensuite les plaquettes dans une solution de glutaraldéhyde à 1% dans l'eau distillée pendant 1 heure à 22°C, puis on les lave avec un tampon phosphate 0,25M de pH 7,5.

On laisse incuber dans une solution d'immunoglobulines (2mg/ml) préalablement dialysée contre du tampon phosphate à 0,25M.

Après incubation pendant 12heures à 4°C, on lave les plaquettes avec un tampon PBS-Tween 20 pendant 1/2 heure à 37°C. Le Tween 20 est une marque commerciale pour un détergent non ionique.

Les plaquettes sont conservées dans une solution de tampon PBS-Tween 20.

On a greffé sur les plaquettes, selon cette méthode, soit des immunoglobulines polyclonales ou monoclonales anti-alphafoetoprotéine, soit des immunoglobulines polyclonales anti-IgE.

### EXEMPLE 2 : Essai avec l'alphafoetoprotéine

On utilise un dispositif analogue à celui de la figure 1.

La fréquence du signal est de 10kHz avec une amplitude de 5 mV.

Le liquide étudié consiste en des solutions tampon contenant des concentrations croissantes d'antigène (alphafoetoprotéine).

Avec des plaquettes de silicium simplement recouvertes de silice, mais non greffées, les tampons salins de type PBS ou glycine, avec ou sans incubation avec des protéines animales, ne donnent pas de variations significatives de la capacité du système quelle que soit la concentration en antigène dans le milieu de mesure et d'incubation.

Par contre, avec une plaquette sur laquelle a été greffée l'anticorps anti-alphaprotéine comme décrit à l'exemple 1, on observe une diminution de la capacité. Pour une tension de polarisation du silicium de l'ordre de + 1,5 Volt par rapport à l'électrode au calomel on a mesuré les capacités suivantes :
- plaquette de référence : C = 5,71.10⁻⁸F
- plaquette porte réactif avec anticorps greffés : C = 4,40.10⁻⁸F.

Un essai avec un tampon ne contenant pas d'antigène a donné la valeur suivante : C = 4,40.10⁻⁸F.

Avec un tampon contenant 10ng/ml d'antigène, on a trouvé C = 4,35.10⁻⁸F.

Avec un tampon contenant 300ng/ml d'antigène, on a trouvé C = 4,27.10⁻⁸F.

### EXEMPLE 3 : Essais de reproduction avec l'alphafoetoprotéine.

On utilise un dispositif analogue à celui de la figure 1.

La plaquette porte un anticorps anti-alphafoetoprotéine couplé par covalence. Concentration de l'antigène : 400ng/ml.

Les conditions de travail sont les mêmes que pour l'exemple 2.

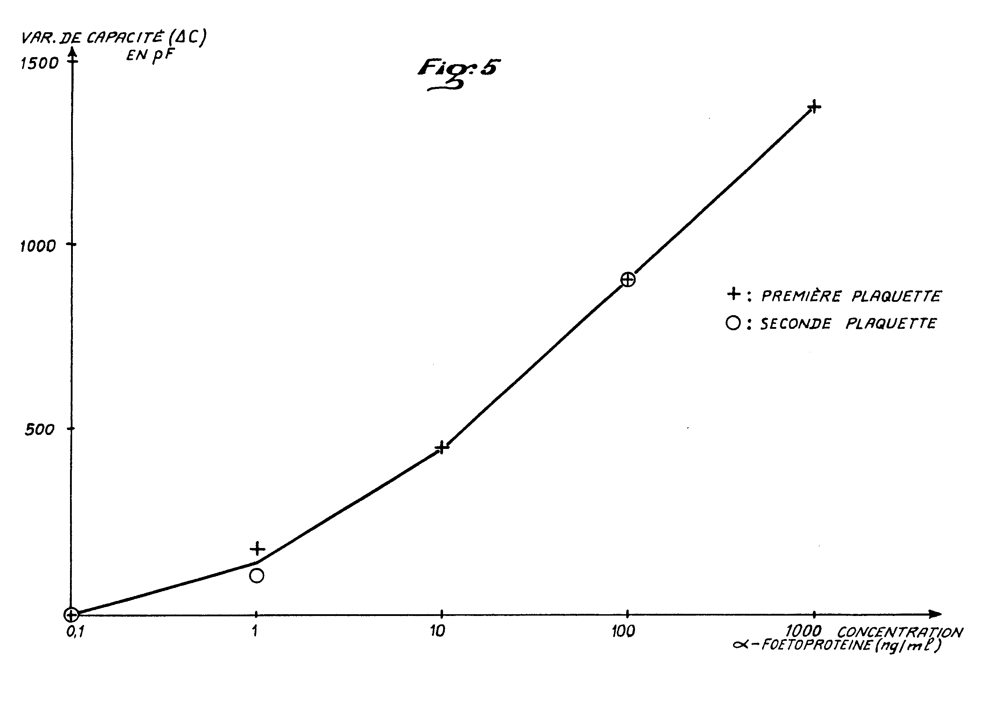

Les résultats obtenus en fonction du temps (fig.5) montrent que la fixation Ag/Ac (polyclonal) s'effectue selon une courbe cinétique du premier ordre ΔC = f (t) linéaire pour des temps courts, ce qui permet des mesures quantitatives.

Les mêmes mesures peuvent être effectuées successivement, après lavage acide, sur la même plaquette activée.

Par ailleurs, deux plaquettes préparées de façon identique donnent des résultats reproductibles (fig.6) en fonction de la concentration de protéine (mesures effectuées après 15 minutes d'incubation).

Dans ce dernier cas, l'anticorps était un anticorps monoclonal.

### EXEMPLE 4 : Essai avec l'IgE humaine

On opère de façon analogue à celle décrite à l'exemple 2.

L'antigène est ici l'IgE humaine testée à diverses concentrations (0 ; 0,2 ; 2 ; 20 ; 200ng/ml).

On utilise des plaquettes sur lesquelles on a greffé des anticorps de chèvre anti-IgE humaines. On a obtenu les résultats suivants :
- tampon sans antigène : C = 6,04.10⁻⁸F.
- tampon contenant 200ng/ml :
   C = 5,88.10⁻⁸F (après 5 minutes d'incubation)
   C = 5,90.10⁻⁸F (après 30 minutes d'incubation).

Après lavage de la plaquette dans un tampon glycine à pH acide pour décrocher les anticorps anti-IgE, on retrouve pratiquement la capacité observée au départ avec le mélange tampon : C = 6,05.10⁻⁸F.

Après une nouvelle incubation de la plaquette ainsi régénérée avec l'antigène, on retrouve le signal caractéristique des sérums positifs en antigène.

Sur une plaquette sur laquelle a été greffée une protéine non spécifique (l'albumine humaine), on n'observe pas de variations significatives du signal après incubation avec des tampons contenant jusqu'à 200ng/ml d'antigènes (anticorps anti-IgE). Les courbes C = f (V) sont pratiquement superposées (V étant la tension de polarisation de la plaquette, par rapport à l'électrode au calomel).

Le signal spécifique est amplifié par l'ajout postérieur d'anticorps anti-IgE dans la phase liquide. L'augmentation d'épaisseur de la couche déposée qui en résulte se traduit par une diminution plus importante de la capacité.

On a en effet observé en effet les valeurs suivantes :
- tampon seul : C = 6,97.10⁻⁸F
- tampon à 200ng/ml d'antigène : C = 6,88.10⁻⁸F
- tampon à 20Ong/ml d'antigène avec ajout postérieur d'anticorps anti-IgE : C = 6,74.10⁻⁸F.

La tension de polarisation du silicium était de + 3 Volt par rapport à l'électrode au calomel.

Le système d'amplification par l'anticorps ajouté en milieu liquide peut être utilisé pour de faibles concentrations d'antigènes (2ng/ml) avec un effet mesurable comme le montre le Tableau 1.

**TABLEAU 1**

| Concentration en antigène (IgE) en ng/ml | Variation spécifique de capacité(anti-IgE) x 10¹¹ Farad | Variation non spécifique (albumine) x 10¹¹ Farad |
|---|---|---|
| 0 | 0 | 0 |
| 2 | 88 | 20 |
| 200 | 125 | 47 |

La sensibilité obtenue sans compensation de la fixation non spécifique est donc de l'ordre de 2ng. Avec une mesure différentielle entre une surface portant une protéine non spécifique telle que de l'albumine et une surface portant un anticorps spécifique, on peut atteindre une sensibilité très supérieure et détecter des concentrations inférieures à 1ng/ml.

Les résultats précédents ont été acquis pour un temps d'incubation et de mesure (5 à 30 minutes) très court par rapport aux autres méthodes de sensibilité comparable. Le signal optimal est généralement obtenu au bout de 5 minutes d'incubation. Bien entendu, la vitesse de réaction est augmentée avec des anticorps greffés hautement spécifiques.

### EXEMPLE 5 : Essai de mesure de capacité et de résistance.

On opère de façon analogue à celle décrite à l'exemple 4 (antigène : IgE humaine).

Les tampons utilisés sont des tampons PBS contenant 0,05% de Tween 20.

Les mesures de capacité ont été effectuées pour une tension de polarisation de la plaque, par rapport à l'électrode au calomel, de + 4 Volt.

Les mesures de résistance ont été effectuée pour une tension de polarisation de la plaque, par rapport à l'électrode au calomel, de + 0,2 Volt.

La fréquence du signal alternatif superposé est de 10 kHz, avec une amplitude de 5mV.

Les résultats sont résumés dans le Tableau 2 ci-après.

**TABLEAU 2**

| plaquette greffée | concentration en IgE | C (nF) | Co - C | R (Ohm) | Ro - R |
|---|---|---|---|---|---|
| Anti-IgE | 0 ng | Co = 72,45 | | Ro = 24,60 | |
| | 1 ng | 71,57 | 0,88 | 21,60 | 3,00 |
| | 100 ng | 71,20 | 1,25 | 20,27 | 4,33 |
| Albumine (référence) | 0 ng | Co = 70,33 | | Ro = 16,04 | |
| | 1 ng | 70,13 | 0,20 | 15,92 | + 0,12 |
| | 100 ng | 69,86 | 0,47 | 16,13 | - 0,09 |

## Revendications

1. Procédé de détection d'une substance biologique dans un milieu liquide conducteur de l'électricité susceptible de la contenir, caractérisé par le fait que l'on met en contact, dans une cellule de mesure, ledit milieu liquide (5, 14, 24) avec une plaque porte-réactif (4, 11, 18) comportant un ligand spécifique de la substance à détecter fixé directement sur une face d'une couche mince en matériau isolant (4a, 11a, 18a) dont l'autre face est fixée sur un support capable de conduire l'électricité, que l'on mesure les composantes C et/ou R de l'impédance électrique du système milieu liquide/couche isolante/support pour une tension donnée, et que l'on compare lesdites composantes à celles d'un système de référence (12,19) analogue au précédent mais exempt de ligand spécifique, une éventuelle diminution de la capacité ou une variation de la résistance, par rapport au système de référence, signifiant la présence de ladite substance biologique dans le milieu liquide étudié.

2. Procédé selon la revendication 1, caractérisé par le fait que le couple ligand-substance biologique est choisi parmi les couples anticorps-antigène, haptène-anticorps, ADN_{c}-ADN, ADN_{c}-ARN, poly dT-ARNₘ eucaryote, enzyme-substrat, enzyme-inhibiteur d'enzyme, lectine-glycoconjugué, marqueur de cellules (ou de microorganismes)-cellules (ou microorganismes), HcG-récepteur tissulaire et T₃ -TBG, et tout couple du type récepteur biologique-(ligand correspondant ou agoniste ou antagoniste).

3. Procédé selon l'une quelconque des revendications précédentes, caractérisé par le fait que, pour augmenter la sensibilité de la détection dans le cas où ladite substance biologique comporte plusieurs sites d'affinité, après avoir mis en contact le liquide étudié avec la plaque porte-réactif, on ajoute audit liquide un second ligand, et que l'on effectue ensuite la mesure de la capacité et/ou de la résistance du système obtenu.

4. Procédé selon la revendication 3, caractérisé par le fait que ledit second ligand est fixé sur une macromolécule telle qu'une protéine ou sur de petites particules individuelles capables de se maintenir en suspension dans ledit milieu liquide.

5. Procédé selon l'une quelconque des revendications précédentes, caractérisé par le fait que ledit ligand est greffé sur la couche isolante par covalence.

6. Procédé selon l'une quelconque des revendications précédentes, caractérisé par le fait que, pour mesurer les composantes de l'impédance électrique, on met une électrode inattaquable (7, 17, 21) en contact avec ledit liquide, on établit une tension alternative entre ladite électrode et ledit support conducteur et on détermine les composantes R et/ou C selon les méthodes usuelles, pour une tension donnée.

7. Plaque porte-réactif destinée à la mise en oeuvre du procédé tel que défini dans l'une quelconque des revendications précédentes, caractérisée par le fait qu'elle comporte un support (4, 11, 12, 18, 19) en matériau capable de conduire l'électricité recouvert sur une de ses faces par une couche mince isolante (4a, 18a) sur laquelle est fixé directement un ligand pour une substance biologique.

8. Plaque porte-réactif selon la revendication 7, caractérisée par le fait que ledit support est en métal et que ladite couche isolante est une couche d'oxyde de ce métal.

9. Plaque porte réactif selon la revendication 8, caractérisée par le fait que ledit métal est l'aluminium ou le tantale.

10. Plaque porte-réactif selon la revendication 7, caractérisée par le fait que ledit support est constitué par un matériau semi-conducteur et qu'il comporte, sur la face opposée à la face recouverte de matériau isolant, une couche métallique destinée à assurer un contact ohmique.

11. Plaque porte-réactif selon la revendication 10, caractérisée par le fait que ledit semi-conducteur est le silicium et que la couche isolante est une couche de silice ou de nitrure de silicium.

12. Plaque porte-réactif selon l'une quelconque des revendications 7 à 11, caractérisée par le fait que ledit ligand est fixé sur la couche isolante par adsorption.

13. Plaque porte-réactif selon l'une quelconque des revendications 7 à 11, caractérisée par le fait que ledit ligand est greffé sur la couche isolante par covalence.

14. Cuve de mesure destinée à mettre en oeuvre le procédé de l'une quelconque des revendications 1 à 6, comprenant une enceinte comportant un espace interne (14, 20) pour l'échantillon liquide, un réceptacle pour une plaque porte-réactif amovible (4, 11, 18), permettant de mettre en communication la face interne, portant la couche isolante munie du ligand, de ladite plaque, avec l'intérieur de l'enceinte, et éventuellement un second réceptacle pour une plaque de référence sans ligand spécifique, des moyens pour assurer l'étanchéité entre l'enceinte et lesdites plaques, une électrode susceptible d'être en contact avec ledit échantillon liquide, et des moyens pour assurer des contacts électriques extérieurs pour l'électrode et pour la face externe des plaques, caracterisée par le fait qu'elle comprend une plaque porte-reactif selon l'une quelconque des revendications 7 à 13.

## Claims

1. Method for detecting a biological substance in an electrically conducting liquid medium capable of containing it, characterised in that the said liquid medium (5, 14, 24) is brought into contact, in a measuring cell, with a reagent-carrier plate (4, 11, 18) containing a specific ligand for the substance to be detected bound directly to one face of a thin layer of insulating material (4a, 11a, 18a), the other face of which is hound to a support capable of conducting electricity; in that the C and/or R components of the electrical impedance of the liquid medium/insulating layer/support system are measured for a given voltage; and in that the said components are compared with those of a reference system (12, 19) similar to the above but devoid of specific ligand, a possible decrease in the capacitance or a change in the resistance, relative to the reference system, denoting the presence of the said biological substance in the liquid medium in question.

2. Method according to Claim 1, characterised in that the biological substance-ligand system is chosen from antibody-antigen, hapten-antibody, cDNA-DNA, cDNA-RNA, poly(dT)-eukaryotic mRNA, enzyme-substrate, enzyme-enzyme inhibitor, lectin-glycoconjugate, cell marker (or microorganism marker)-cells (or microorganisms), HcG-tissue receptor and T₃-TBG systems, and any system of the biological receptor-(agonist or antagonist or corresponding ligand) type.

3. Method according to either of the preceding claims, characterised in that, to increase the sensitivity of the detection in cases where the said biological substance contains several affinity sites, after the liquid in question has been brought into contact with the reagent-carrier plate, a second ligand is added to the said liquid, and in that the measurement of the capacitance and/or the resistance of the system obtained is then performed.

4. Method according to Claim 3, characterised in that the said second ligand is bound to a macromolecule such as a protein or to individual small particles capable of being maintained in suspension in the said liquid medium.

5. Method according to any one of the preceding claims, characterised in that the said ligand is grafted covalently onto the insulating layer.

6. Method according to any one of the preceding claims, characterised in that, to measure the components of the electrical impudence, an electrode incapable of being attacked (7, 17, 21) is brought into contact with the said liquid, an a.c. voltage is established between the said electrode and the said conducting support and the R and/or C components are determined according to the usual methods, for a given voltage.

7. Reagent-carrier plate designed for carrying out the method as defined in any one of the preceding claims, characterised in that it contains a support (4, 11, 12, 18, 19) made of a material capable of conducting electricity, covered on one of its faces with a thin insulating layer (4a, 18a) to which a ligand for a biological substance is bound directly.

8. Reagent-carrier plate according to Claim 7, characterised in that the said support is made of metal and that the said insulating layer is a layer of oxide of this metal.

9. Reagent-carrier plate according to Claim 8, characterised in that the said metal is aluminium or tantalum.

10. Reagent-carrier plate according to Claim 7, characterised in that the said support consists of a semiconducting material and that it contains, on the face opposite the face covered with insulating material, a metallic layer designed to provide for ohmic contact.

11. Regent-carrier plate according to Claim 10, characterised in that the said semiconductor is silicon and that the insulating layer is a layer of silica or of silicon nitride.

12. Reagent-carrier plate according to any one of Claims 7 to 11, characterised in that the said ligand is bound to the insulating layer by adsorption.

13. Reagent-carrier plate according to any one of Claims 7 to 11, characterised in that the said ligand is grafted covalently onto the insulating layer.

14. Measuring call designed for carrying out the method of any one of Claims 1 to 6, comprising an enclosure containing an internal apace (14, 20) for the liquid sample, a receptacle for a removable reagent-carrier plate (4, 11, 18), enabling the inner face, bearing the insulating layer equipped with the ligand, of the said plate to be brought into communication with the inside of the enclosure, and optionally a second receptacle for a referance plate without a specific ligand, means for providing for sealing between the enclosure and the said plates, an electrode capable of being in contact with the said liquid sample, and means far providing for external electrical contacts for the electrode and for the outer face of the plates, characterised in that it comprises a reagent-carrier plate according to any one of Claims 7 to 13.

## Patentansprüche

1. Verfahren zum Aufspüren einer biologischen Substanz in einem flüssigen Milieu, das in der Lage ist, einen elektrischen Leiter zu enthalten, **gekennzeichnet durch** die Tatsache, dass man das flüssige Milieu (5, 14, 24) innerhalb einer Meßzelle mit einer ein reagierendes Mittel tragenden Platte (4, 11, 18) in Kontakt bringt, welche einen spezifischen Liganden der aufzuspürenden Substanz trägt, der direkt auf einer Oberfläche eines sehr kleinen Bettes aus einem isolierenden Material (4a, 11a, 18a) befestigt ist, während die andere Fläche auf einem Träger befestigt ist, der in der Lage ist, den elektrischen Strom zu leiten, daß man die Größen C und/oder R der elektrischen Impedanz des Systems aus flüssigem Milieu/isolierendem Bett / Träger für eine gegebene Spannung mißt und daß man die genannten Größen mit denjenigen eines Referenzsystems (12, 19) vergleicht, das dem vorgenannten System analog ist mit der Ausnahme des spezifischen Liganden, wobei eine eventuelle Verminderung der Kapazität oder eine Abweichung des Widerstandes hinsichtlich des Referenzsystems gemessen wird, welche die Gegenwart der biologischen Substanz im untersuchten flüssigen Milieu anzeigt.

2. Verfahren nach Anspruch 1, **gekennzeichnet durch** die Tatsache, dass das Paar aus Ligand und biologischer Substanz ausgewählt ist aus den Paarungen Antikörper-Antigen, Hapten-Antikörper, ADN_{c}-ADN, ADN_{c}-ARN, Poly-dT-ARNₘ Eucaryot, Enzym-Substrat, Enzyminhibitor, Lectin-Glycoconjugiertes Substrat, Zellmarkierer (oder von Mikroorganismen) - Zellen (oder Mikroorganismen) HcG - Geweberezeptor und T₃-TBG sowie jede weitere Paarung vom Typ biologischer Rezeptor - (entprechender Ligand oder Agonist oder Antagonist).

3. Verfahren nach den vorhergehenden Ansprüche **gekennzeichnet durch** die Tatsache, dass man zur Erhöhung der Empfindlichkeit der Aufspürung in dem Fall, dass die biologische Substanz mehrere Affinitätsstellen besitzt, nachdem man die Untersuchungsflüssigkeit mit der das Reaktionsmittel tragende Platte in Kontakt gebracht hat, zu der Flüssigkeit einen zweiten Liganden zusetzt und dass man erneut das Messen von Kapazität und/oder Widerstand des erhaltenen Systems durchführt.

4. Verfahren nach Anspruch 3, **gekennzeichnet durch** die Tatsache, dass der zweite Ligand auf einem Makromolekül fixiert ist, wie ein Protein oder auf kleine einzelne Partikel, die in der Lage sind, in Suspension in dem flüssigen Milieu gehalten zu werden.

5. Verfahren nach den vorhergehenden Ansprüchen, **gekennzeichnet durch** die Tatsache, daß der Ligand auf das isolierende Bett durch kovalente Bindungen aufgepfropft ist.

6. Verfahren nach den vorhergehenden Ansprüchen, **gekennzeichnet durch** die Tatsache, dass man zum Messen der Größen der elektrischen Impedanz eine nicht angreifbare Elektrode, (7, 17, 21) in Kontakt mit der genannten Flüssigkeit bringt, dass man eine alternative Spannung zwischen dieser Elektrode und dem leitfähigen Träger einrichtet und dass man die Größen R und/oder C mit den üblichen Methoden für eine gegebene Spannung bestimmt.

7. Platte als Träger für ein Reaktionsmittel zur Verwendung in dem Verfahren, wie es in den vorstehenden Ansprüchen definiert ist, **gekennzeichnet durch** die Tatsache, dass sie aus einem Träger (4, 11, 12, 18, 19) aus einem Material besteht, das in der Lage ist, die Elektrizität zu leiten, wobei eine der Flächen mit einem sehr kleinen Isolierbett (4a, 18a) bedeckt ist, auf welchem unmittelbar ein Ligand für eine biologische Substanz fixiert ist.

8. Platte als Träger für ein Reaktionsmittel nach Anspruch 7**, gekennzeichnet durch** die Tatsache, dass dieser Träger ein Metall ist und dass das Isolierbett ein Bett aus einem Oxyd dieses Metalles ist.

9. Platte als Träger für ein Reaktionsmittel nach Anspruch 8, **gekennzeichnet durch** die Tatsache, dass das Metall Aluminium oder Tantal ist.

10. Platte als Träger für ein Reaktionsmittel nach Anspruch 7, **gekennzeichnet durch** die Tatsache, dass dieser Träger aus einem halbleitenden Material besteht und dass es auf der Fläche, die der mit dem isolierenden Material bedeckten Fläche gegenüber liegt, mit einem Metallbett beschichtet ist, das einen Ohm'schen Kontakt sicherstellt.

11. Platte als Träger für ein Reaktionsmittel nach Anspruch 10, **gekennzeichnet durch** die Tatsache, dass der Halbleiter aus Silicium ist und dass das Isolierbett ein Bett aus Kieselsäure und Siliciumnitrid ist.

12. Platte als Träger für ein Reaktionsmittel nach einem der Ansprüche 7 bis 11, **gekennzeichnet durch** die Tatsache, dass der Ligand auf dem Isolierbett durch Adsorption fixiert ist.

13. Platte als Träger für ein Reaktionsmittel nach einem der Ansprüche 7 bis 11, **gekennzeichnet durch** die Tatsache, dass der Ligand auf das Isolierbett durch kovalente Bindungen aufgepfropft ist.

14. Meßküvette, bestimmt für das Messen nach dem Verfahren der Ansprüche 1 bis 6, die einen umflossenen Raum darstellt mit einem Innenraum (14, 20) zur Aufnahme der Flüssigkeit, einem Behälter für eine Platte, die ein Reaktionsmittel trägt und auswechselbar ist (4, 11, 18), die es gestattet, eine Messung in Verbindung mit der Innenfläche durchzuführen, welche ein Isolierbett trägt, auf welchem ein Ligand auf der Platte befestigtist, mit einem Innenraum des umschlossenen Raums und wobei gegebenenfalls ein zweiter Aufnahmebehälter für eine Referenzplatte ohne spezifischen Liganden vorgesehen ist, und daß Mittel vorgesehen sind, um die Dichtigkeit zwischen dem umschlossenen Raum und den Platten sicherzustellen, und eine Elektrode vorgesehen ist, mit deren Hilfe der Kontakt mit der flüssigen Probe hergestellt werden kann und Mittel vorgesehen sind, um die elektrischen Kontakte nach außen zwischen Elektrode und Außenfläche der Platten sicherzustellen. **dadurch gekennzeichnet,** daß sie eine Platte als Träger für ein Reaktionsmittel nach einem der Ansprüche 7 bis 13 enthält.
